(19) Europäisches Patentamt
European Patent Office
Office européen des brevets

(11) Veröffentlichungsnummer : **0 498 765 A1**

(12) **EUROPÄISCHE PATENTANMELDUNG**

(21) Anmeldenummer : **92810057.7**

(51) Int. Cl.[5] : **C07C 319/14,** C07C 319/20

(22) Anmeldetag : **27.01.92**

(30) Priorität : **04.02.91 CH 327/91**

(43) Veröffentlichungstag der Anmeldung :
**12.08.92 Patentblatt 92/33**

(84) Benannte Vertragsstaaten :
**BE DE ES FR GB IT NL**

(71) Anmelder : **CIBA-GEIGY AG**
**Klybeckstrasse 141**
**CH-4002 Basel (CH)**

(72) Erfinder : **Drewes, Rolf**
**Am Buchacker 1**
**W-6145 Lindenfels (DE)**
Erfinder : **Friedrich, Hans-Helmut**
**Am Rauhenstein 8**
**W-6147 Lautertal 2 (DE)**

(54) **Verfahren zur Herstellung von Alkyl- und Arylthiocarbonsäuren und deren Estern.**

(57) Es wird ein Verfahren zur Herstellung von Alkylthio- oder Arylthiocarbonsäuren bzw. deren Alkylestern der Formel I

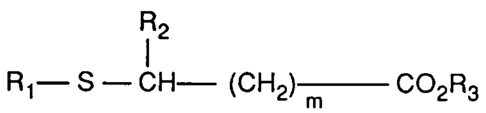
(I)

beschrieben, worin $R_1$ $C_{1-20}$Alkyl oder mit $C_{1-4}$Alkoxy oder $C_{1-4}$Alkylthio substituiertes $C_{1-20}$Alkyl, $C_{5-12}$Cycloalkyl, Phenyl, mit $C_{1-4}$Alkylo oder $C_{1-4}$Alkoxy substituiertes Phenyl oder Phenyl $C_{1-4}$Alkyl bedeutet, $R_2$ Wasserstoff, $C_{1-12}$Alkyl oder mit $C_{1-4}$Alkoxy oder $C_{1-4}$Alkylthio substituiertes $C_{1-12}$Alkyl, Phenyl oder mit $C_{1-4}$Alkyl oder $C_{1-4}$Alkoxy substituiertes Phenyl ist, $R_3$ Wasserstoff oder $C_{1-6}$Alkyl und m eine Zahl von 1 bis 4 bedeutet, dadurch gekennzeichnet, daß (A) ein Mercaptan $R_1SH$ in einem aprotischen Lösungsmittel mit Siedepunkt oberhalb 80°C mittels eines Alkalimetall-$C_{1-4}$-alkoholats oder -hydroxids als Base in sein Alkalisalz übergeführt wird, (B) mit einem cyclischen, 4 bis 7 gliedrigen, Lacton der Formel II

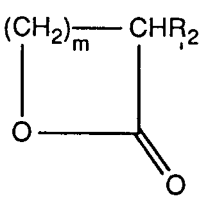
(II)

umgesetzt wird, und das entstandene Salz (C1) durch Ansäuern mit Mineralsäuren in eine Säure der Formel I ($R_3$ = H) übergeführt und isoliert wird bzw. (C2) mit einem Alkohol $R_3OH$ zum Ester der Formel I ($R_3$ = $C_{1-0}$Alkyl) verestert wird, wobei alle Stufen ohne Isolierung von Zwischenprodukten im in Stufe (A) eingesetzten Lösungsmittel durchgeführt werden. Die Ester der Formel I ($R_3$ = $C_{1-0}$Alkyl) können als Zwischenprodukte bei der Herstellung von β-Diketonen eingesetzt werden, die als Stabilisatoren für chlorhaltige Polymere von Nutzen sind.
Die Säuren der Formel I ($R_3$ = H) finden z.B. Verwendung als Korrosionsinhibitoren und Verschleißschutzadditive in Schmierstoffen und anderen funktionellen Flüssigkeiten.

EP 0 498 765 A1

Die Erfindung betrifft ein Verfrahen zur Herstellung von Alkyl- und Arylthiocarbonsäuren und deren Estern durch Umsetzung von Lactonen mit Thiolaten und gegebenenfalls direkte Weiterreaktion mit einem niederen Alkohol sowie ein Verfahren zur Herstellung von β-Diketonen aus den so erhaltenen Estern,

1955 wurde von Reppe ein Verfahren zur Herstellung von γ-Methylmercaptobuttersäure durch Reaktion von γ-Butyrolacton mit Natriumthiomethylat in Methanol angegeben [Annalen 596, 163 (1955)]. Die Ausbeute betrug 56%. Node et al. veröffentlichten 1981 ein weiteres Verfahren, bei dem das Lacton mit Lewis-Säuren aktiviert wird und dann mit Alkylsulfid umgesetzt wird [J. Org. Chem. 46, 5163 (1981)]. Die DE-A- 22 233 90 gibt ein Verfahren an, bei dem Thiole unter Stickstoff mit Natriumethylat und γ-Butyrolactonen umgesetzt werden. Ein weiteres Verfahren ist aus N. Arsenescu, I.I. Badilescu, Revista de Chimie 24, 961 (1963) bekannt: (Substituiertes) Thiophenol wird in absolutem Ethanol mit Natriumalkoholat und γ-Butyrolacton zur (am Phenylring substituierten) γ-Phenylmercaptobuttersäure umgesetzt. Der EP-A-0 245 231 ist ferner ein Verfahren zur Herstellung der Salze von Alkylthioalkansäuren durch Reaktion eines Alkalialkylthiolats mit einem Lacton in Gegenwart eines polaren aprotischen, organischen Lösungsmittels, z. B. Dimethylformamid (DMF), Hexamethylphosphorsäuretrisamid (HMPTA), Dimethylsulfoxid (DMSO), Tetramethylharnstoff (TMU), bevorzugt jedoch Pyridin oder Alkylpyridine, zu entnehmen.

Diese Verfahren befriedigen noch nicht, da sie aus verschiedenen Gründen, wie z.B. geringe Ausbeute oder teure Katalysatoren, eine großtechnische Anwendung zumindest erschweren. Daher besteht weiterhin ein Bedarf nach Verfahren, mit denen Alkyl- bzw. Arylmercaptocarbonsäuren und deren Ester unter einfachen Bedingungen und in guter Ausbeute hergestellt werden können.

Es wurde nun überraschenderweise gefunden, daß man mit einer Kombination bestimmter Verfahrensmaßnahmen Bedingungen schaffen kann, bei denen man direkt und ohne Isolierung von Zwischenprodukten zu Alkyl- bzw. Arylmercaptocarbonsäureestern kommt. Unterläßt man dabei den Veresterungsschritt, so erhält man auch in guter Ausbeute die entsprechende Säure. Man kann dabei sowohl auf die relativ teuren und wenig umweltfreundlichen Lewis-Säuren verzichten als auch hohe Ausbeuten (bis über 90% Ester) erzielen.

Die Erfindung besteht somit in einem Verfahren zur Herstellung von Alkylthio- oder Arylthiocarbonsäuren bzw. deren Alkylestern der Formel I

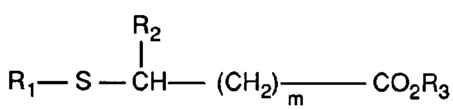

(I),

worin

$R_1$ $C_{1-20}$Alkyl oder mit $C_{1-4}$Alkoxy oder $C_{1-4}$Alkylthio substituiertes $C_{1-20}$Alkyl, $C_{5-12}$Cycloalkyl, Phenyl, mit $C_{1-4}$Alkyl oder $C_{1-4}$Alkoxy substituiertes Phenyl oder Phenyl-$C_{1-4}$Alkyl bedeutet,

$R_2$ Wasserstoff, $C_{1-12}$Alkyl oder mit $C_{1-4}$Alkoxy oder $C_{1-4}$Alkylthio substituiertes $C_{1-12}$Alkyl, Phenyl oder mit $C_{1-4}$Alkyl oder $C_{1-4}$Alkoxy substituiertes Phenyl ist, $R_3$ Wasserstoff oder $C_{1-6}$Alkyl und m eine Zahl von 1 bis 4 bedeuten, dadurch gekennzeichnet, daß

(A) ein Mercaptan $R_1SH$ in einem aprotischen Lösungsmittel mit Siedepunkt oberhalb 80°C mittels eines Alkalimetall-$C_{1-4}$-alkoholats oder -hydroxids als Base in sein Alkalisalz übergeführt wird,

(B) mit einem cyclischen, 4- bis 7-gliedrigen Lacton der Formel II

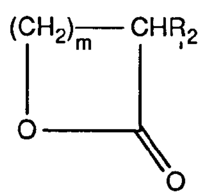

(II)

umgesetzt wird,

und das entstandene Salz

(C1) durch Ansäuern mit starken Mineralsäuren in eine Säure der Formel I ($R_3$ = H) übergeführt und diese isoliert wird bzw.

(C2) mit einem Alkohol $R_3OH$ zum Ester der Formel I ($R_3$ = $C_{1-6}$Alkyl) verestert wird, wobei alle Stufen ohne Isolierung von Zwischenprodukten im in Stufe (A) eingesetzten Lösungsmittel durchgeführt werden.

Bedeuten in obigen Formeln $R_1$ und $R_2$ $C_{1-20}$-Alkyl, so handelt es sich dabei um verzweigte oder unverzweigte Reste, z.B. um Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl, 1-Methylundecyl oder Eicosyl. Bedeuten diese Reste Gruppen mit einer geringeren Zahl von C-Atomen, so sind entsprechende Beispiele ebenfalls obiger Liste zu entnehmen. Die Alkylgruppen können ihrerseits noch mit $C_{1-4}$Alkoxy- oder $C_{1-4}$Alkylthiogruppen, also -O-$C_{1-4}$Alkyl oder -S-$C_{1-4}$Alkyl, substituiert sein, wobei der Alkylrest die Bedeutung von Methyl, Ethyl, Propyl, Isopropyl, n-, i-, oder t-Butyl annimmt. Dieselben Bedeutungen kann $R_3$ als $C_{1-6}$Alkyl annehmen sowie zusätzlich noch z.B. n-Pentyl Isopentyl, 2-Methylpentyl oder Hexyl. Bevorzugt ist $R_2$ Wasserstoff oder $C_{1-4}$Alkyl, $R_3$ ist bevorzugt Methyl oder Ethyl, insbesondere Methyl. Als $C_{5-12}$-Cycloalkyl bedeutet $R_1$ beispielsweise Cyclopentyl, Cyclohexyl, Cycloheptyl, Cyclooctyl, Cyclodecyl und Cyclododecyl. Bevorzugt sind Cyclopentyl, Cyclohexyl und Cycloheptyl, besonders bevorzugt ist Cyclohexyl. Ist $R_1$ Phenyl-$C_{1-4}$-alkyl, so sind Beispiele dafür Benzyl, Phenethyl, 3-Phenylpropyl, α-Methylbenzyl und α,α-Dimethylbenzyl. Bevorzugt ist Benzyl.

$R_1$ ist bevorzugt unsubstituiertes $C_{1-18}$-, insbesondere $C_{4-12}$Alkyl Phenyl, Benzyl oder $C_{5-7}$Cycloalkyl. $R_2$ ist insbesondere Wasserstoff oder $C_{1-4}$Alkyl, z.B. Wasserstoff oder Methyl, vor allem Wasserstoff; m steht bevorzugt für 2.

Wie eingangs bereits angedeutet kommt es bei dem erfindungsgemäßen Verfahren vor allem entscheidend auf die richtige Auswahl des Lösungsmittels an. Die Kriterien, nach denen dies geschieht, unterscheiden sich z.T. von den bisher im Stand der Technik angebotenen und bilden einen integralen Bestandteil der Erfindung. In EP-A-0 245 231 wird darauf hingewiesen, daß das Lösungsmittel für die erfolgreiche Durchführung des dort beschriebenen Verfahrens aprotisch und polar sein muß, d.h. ein nennenswertes Dipolmoment ($D > 1$ Debye) besitzen muß. Außerdem sollte es eine Dielektrizitätskonstante $\varepsilon$ von möglichst über 10 haben. Im Gegensatz dazu wurde nun gefunden, daß es nicht auf die Polarität des Lösungsmittels ankommt, sondern darauf, daß der Siedepunkt (b.p.) ausreichend hoch ist, d. h. über 80°C liegt. Denn nur bei genügend hohen Temperaturen liegen die Reaktionsteilnehmer überhaupt in homogener Phase vor bzw. werden die anfallenden Salze in ausreichender Menge gelöst. Bevorzugt sind als Lösungsmittel lineare oder cyclische Ether, lineare oder cyclische Amide oder aromatische Kohlenwasserstoffe, die in diesem Siedebereich liegen.

Als besonders bevorzugte Lösungsmittel gelten demzufolge Dioxan ($D = 0$; $\varepsilon = 2{,}209$; b.p. 101°C) Diethylenglykoldimethylether, im folgenden abgekürzt: Diglyme ($D = 1{,}97$; $\varepsilon = 5{,}79$; b.p. 162°C), Dibutylether ($D = 1{,}17$; b.p. 142-3°C), Dimethylformamid ($D = 3{,}82$, b.p. 153°C) N-Methylpyrrolidon ($D = 4{,}09$, $\varepsilon = 32{,}2$; b.p. 202°C), Mesitylen ($D = 0$, $\varepsilon = 2{,}27$, b.p. 165°C) sowie die isomeren Xylole als Mischung oder einzeln: z.B. o-Xylol ($D = 0{,}62$, $\varepsilon = 2{,}57$, b.p. 144°C), p-Xylol ($D = 0$, $\varepsilon = 2{,}27$, b.p. 138°C). Weiterhin gültig bleibt als Kriterium für die Auswahl des Lösungsmittels die Forderung, daß es aprotisch sein muß. Die Reaktionstemperaturen liegen in der Stufe (B) zweckmäßig zwischen 80°C und dem Siedepunkt des gewählten Lösungsmittels, bevorzugt zwischen 80 und 130°C, besonders bevorzugt zwischen 90 und 120°C.

Die Ausgangsstoffe Lacton und Mercaptan werden vorteilhaft in aequimolaren Mengen eingesetzt. Im Schritt (C2) wird der Alkohol zweckmäßig im Überschuß eingesetzt, der beispielsweise das Ein- bis Zwanzigfache, bevorzugt das Anderthalb- bis Zehnfache der aequimolaren Menge sein kann.

Um die besonders vorteilhafte Durchführung des Verfahrens zu gewährleisten und es insbesondere zu ermöglichen, ohne Zwischenisolierung der Säure ($R_3$=H) in hohen Ausbeuten direkt zu den entsprechenden Estern zu gelangen, ist es ferner erforderlich, in der Stufe (A) ein Alkalimetallalkoholat oder -hydroxid als Base einzusetzen. Besonders bevorzugt sind die Basen NaOH, KOH, Natrium- oder Kaliummethylat, -ethylat oder -butylat, insbesondere die genannten Alkoholate..

Bevorzugt ist die Ausführungsform des beschriebenen Verfahrens für Verbindungen der Formel I, bei denen $R_1$ $C_{1-18}$Alkyl, $C_{5-7}$Cycloalkyl, Phenyl oder Benzyl ist, $R_2$ Wasserstoff oder $C_{1-4}$Alkyl ist und m für 2 steht. Besonders bevorzugt werden mit dem erfindungsgemäßen Verfahren solche Verbindungen der Formel I hergestellt, worin $R_1$ $C_9$-$C_{12}$-Alkyl, $R_2$ Wasserstoff, $R_3$ Methyl sind und m die Zahl 3 bedeutet, ferner auch solche, worin $R_1$ $C_{12}$-Alkyl ist, $R_2$ und $R_3$ Wasserstoff bedeuten und m die Zahl 2 ist.

Ebenfalls besonders bevorzugt bezieht sich das erfindungsgemäße Verfahren auf die Herstellung der Ester, d.h. in Formel I ist $R_3$ $C_{1-6}$Alkyl.

Dabei kann die Veresterung in bekannter Weise, z.B. sauer oder alkalisch, katalysiert werden oder unter Entfernung von Wasser mittels azeotroper Destillation, Dehydratisierungsagentien wie Dicyclohexylcarbodiimid oder Molekularsieb ablaufen. Sie findet jedoch bevorzugt unter sauren Bedingungen statt, z. B. in Gegenwart von Mineralsäuren wie HCl, $H_3PO_4$ oder $H_2SO_4$, ganz besonders bevorzugt mit Schwefelsäure als Katalysator. Die Reaktionsmischung wird nach der Veresterung zweckmäßig mit geeigneten Basen, vorzugsweise Soda, Pottasche oder Natrium- oder Kaliumhydrogencarbonat neutralisiert, und das Produkt wird z.B. durch Extraktion und Abziehen des Extraktionsmittels isoliert.

Da die erfindungsgemäßen Verbindungen der Formel I mit $R_3$ = $C_{1-6}$Alkyl zur Weiterverarbeitung zu β-Diketonen eingesetzt werden können (vgl. EP-A-0 307 358), eröffnet ihre Zugänglichkeit auch den Weg zu Verbindungen der Formel III

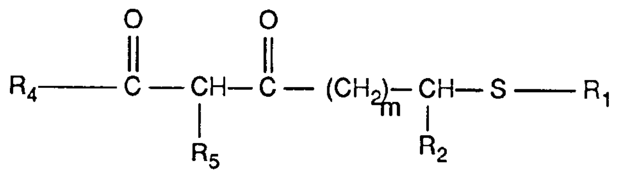
(III),

worin $R_4$ für $C_1$-$C_{20}$-Alkyl, Phenyl, durch Halogen, Hydroxy, $NO_2$, $C_1$-$C_4$-Alkyl und/oder $C_1$-$C_4$-Alkoxy substituiertes Phenyl, $C_7$-$C_9$-Phenylalkyl oder für einen Rest der Formel V steht,

-A-X-$R_6$ (V)

wobei
A $C_1$-$C_{12}$-Alkylen, Phenylen, durch Halogen, Hydroxy, $NO_2$, $C_1$-$C_4$-Alkyl und/oder $C_1$-$C_4$-Alkoxy substituiertes Phenylen oder durch Hydroxy, Halogen oder/und Alkoxy substituiertes $C_1$-$C_{12}$-Alkylen bedeutet,
X für Sauerstoff oder Schwefel steht und
$R_6$ Wasserstoff, $C_1$-$C_{18}$-Alkyl, Phenyl, durch Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl bedeutet und $R_5$ Wasserstoff, $C_1$-$C_{20}$-Alkyl, Phenyl, durch Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl darstellt.
Die Erfindung betrifft daher auch ein Verfahren zur Herstellung von β-Diketonen der Formel III, dadurch gekennzeichnet daß
(D) die erfindungsgemäß hergestellten Verbindungen der Formel I mit $R_3$ = $C_{1-6}$Alkyl mittels herkömmlicher Kondensationsmethoden mit Ketonen der Formel IV

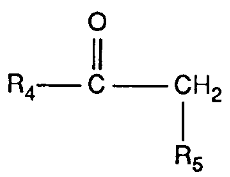
(IV)

in Gegenwart einer Base umgesetzt werden. Es handelt sich dabei um eine Claisen Kondensation, wie sie beispielsweise in J. March, Advanced Organic Chemistry, J. Wiley & Sons (1985), S. 437-39, 835 beschrieben ist.

Ist $R_4$ substituiertes Phenyl, enthält letzteres z.B. 1 bis 3, insbesondere 1 oder 2 Substituenten, vorzugsweise einen Substituenten. $R_4$ als ($C_1$-$C_4$-Alkyl)-phenyl kann beispielsweise für mit 1-3, insbesondere 1 oder 2 Alkylgruppen, vor allem mit Methylgruppen substituiertes Phenyl stehen. Beispiele dafür sind Tolyl, Xylyl oder Mesityl.
$R_4$ als durch Halogen substituiertes Phenyl kann beispielsweise einen ein- oder mehrfach durch Fluor, Chlor oder Brom, insbesondere Chlor oder Brom substituierten Phenylring bedeuten, wie z.B. Chlorphenyl oder Dichlorphenyl.
$C_1$-$C_4$-Alkoxy bedeutet beispielsweise Methoxy, Ethoxy, Propyloxy oder Butoxy, ein entsprechend substituiertes Phenyl ist beispielsweise Methoxyphenyl.
$R_4$ als $C_7$-$C_9$-Phenylalkyl steht z.B. für Benzyl, Phenylethyl, α-Methylbenzyl, 3-Phenylpropyl oder α,α-Dimethylbenzyl, bevorzugt ist Benzyl. Bevorzugt ist $R_4$ $C_1$-$C_{20}$-Alkyl, Phenyl, ($C_1$-$C_4$-Alkyl)phenyl oder -A-X-$R_6$.

A als $C_1$-$C_{12}$-Alkylen kann beispielsweise ein lineares oder verzweigtes, vorzugsweise lineares Alkylen bedeuten. Beispiele für solche Reste ergeben sich aus den obigen Beispielen für Alkyl als $R_1$ und $R_2$ bis zur entsprechenden Anzahl der C-Atome durch Anfügen des Suffixes -en. Bevorzugt ist $C_1$-$C_6$-Alkylen, insbesondere n-Propylen oder n-Pentylen.

$R_6$ als $C_1$-$C_{18}$-Alkyl kann z.B. für lineares oder verzweigtes Alkyl stehen, wie beispielsweise für $R_1$ und $R_2$ beschrieben, bis zur entsprechenden Anzahl der C-Atome.
$R_6$ als substituiertes Phenyl oder $C_7$-$C_9$-Phenylalkyl kann die gleichen Bedeutungsmöglichkeiten haben wie für $R_1$ und $R_2$ beschrieben.
$R_6$ ist bevorzugt Wasserstoff, $C_1$-$C_{18}$-Alkyl, Phenyl oder ($C_1$-$C_4$-Alkyl)-phenyl.
A als gegebenenfalls substituiertes Phenylen ist vorzugsweise o- oder p-Phenylen, insbesondere unsubstitu-

iertes Phenylen.

$R_5$ als $C_1$-$C_{20}$-Alkyl, substituiertes Phenyl oder $C_7$-$C_9$-Phenylalkyl kann die gleichen Bedeutungsmöglichkeiten haben wie für $R_1$ und $R_2$ beschrieben, bevorzugt ist $C_1$-$C_4$-Alkyl.

$R_5$ steht bevorzugt für Wasserstoff oder $C_1$-$C_4$-Alkyl, ganz besonders bevorzugt ist $R_5$ Wasserstoff.

Bevorzugt nutzt man Schritt (D) zur Herstellung von β-Diketonen der Formel III, worin $R_4$ $C_1$-$C_{20}$-Alkyl, Phenyl, ($C_1$-$C_4$-Alkyl)-phenyl oder einen Rest der Formel V bedeutet,
A für $C_1$-$C_6$-Alkylen steht,
$R_5$ Wasserstoff, $C_1$-$C_{18}$-Alkyl, Phenyl oder ($C_1$-$C_4$-Alkyl)-phenyl darstellt und
$R_5$ für Wasserstoff und $C_1$-$C_4$-Alkyl steht.

Es folgt eine Erläuterung von bevorzugten Ausführungsformen der einzelnen Verfahrensschritte:

Schritt (A): Zunächst wird die Base, vorzugsweise ein Alkalimetallalkoholat, hergestellt. Das kann durch Suspendieren von NaOH- oder KOH-Pulver im gewählten Lösungsmittel, wobei das Neutralisationswasser zweckmäßig zu entfernen ist, oder vorzugsweise durch Auflösen des Alkalimetalls in dem wasserfreien Alkohol und anschließende Zugabe des gewählten Lösungsmittels geschehen. Das Mercaptan $R_1SH$ wird sodann, vorzugsweise unter Rühren, zugegeben. Die Temperatur bei diesem Schritt kann beispielsweise zwischen 10 und 60 °C, vorzugsweise zwischen 20 und 40°C, insbesondere bei Raumtemperatur liegen.

Schritt (B): Das Lacton der Formel II wird vorzugsweise im gleichen Lösungsmittel gelöst und zu der aus Schritt (A) erhaltenen, gegebenenfalls erwärmten Lösung zudosiert. In einer besonderen Ausführungsform erfolgt diese Zugabe mit einer unterhalb des Flüssigkeitsspiegels angebrachten Dosiereinrichtung. Dies sollte je nach Größe des Ansatzes ab ca. 1/2 Stunde dauern. Die Mischung wird noch eine weitere Zeit bei erhöhter Temperatur gehalten.

Schritt (C1): Die Säure kann nun mit geeigneten wäßrigen Mineralsäuren (z.B. HCl, $H_2SO_4$, $H_3PO_4$) ausgefällt und isoliert werden.

Alternativ zu (C1) wird Schritt (C2) durchgeführt und insbesondere säurekatalysiert (z.B. mit HCl, $H_2SO_4$, $H_3PO_4$, bevorzugt mit ca 0,8 Aequivalenten $H_2SO_4$) mit einem Überschuß des gewünschten Alkohols umgesetzt, neutralisiert, der Alkohol nach Möglichkeit abgezogen und die gegebenenfalls verdünnte wäßrige Phase mit organischen Lösungsmitteln extrahiert (z.B. Methylenchlorid, Dibutylether, Hexan, Toluol, Xylol, Petrolether). Durch Vereinigen mit der organischen Phase und nach Abziehen des Extraktionsmittels erhält man das Rohprodukt des Esters, das mit geeigneten Mitteln (z.B Chromatographie, fraktionierte Destillation) gereinigt wird. In einer besonders bevorzugten Ausführungsform des Schrittes (C2) verdünnt man die Mineralsäure mit einem Überschuß des niederen Alkohols, der dem gewünschten Ester entspricht, und tropft diese Mischung so zu der Reaktionsmischung (B), daß ein Rückfluß aufrechterhalten wird. Nach weiterem, mehrstündigem Rühren unter Rückfluß wird ein Teil der überschüssigen Säure neutralisiert, eventuell mit Natron- oder Kalilauge, vorzugsweise jedoch mit Soda, Pottasche oder Natrium- oder Kaliumhydrogencarbonat. Nach Abziehen des überschüssigen niederen Alkohols wird auf Raumtemperatur abgekühlt und mit Wasser vermischt. Die wäßrige Phase wird mehrmals mit Ether extrahiert und mit der organischen Phase vereinigt, die dann mittels fraktionierter Destillation gereinigt wird.

Schritt (D): Die Claisen Kondensation bedarf hier als Standardmethode der organischen Chemie an sich keiner näheren Erläuterung. Die konkreten Verfahrensparameter wie Reaktionszeit, Druck und Temperatur, Molverhältnisse können in weiten Grenzen schwanken. Es ist ferner eine Vielzahl von geeigneten Lösungsmitteln denkbar. Dennoch seien hier beispielhaft einige Parameter spezifiziert:

Zweckmäßig ist die Durchführung des erfindungsgemäßen Schrittes (D) bei Temperaturen zwischen -20 und +70°C. Bevorzugt liegen die Reaktionstemperaturen z.B. zwischen -5 und +40°C.

Die Esterkomponente und/oder die Base setzt man zweckmäßig in einer Menge von 0,5-1,5 Mol, vorzugsweise 0,65-1,25 Mol, insbesondere 0,9-1,2 Mol, bezogen auf 1 Mol Keton, ein.

Als Basen eignen sich Erdalkali- und Alkalimetallhydride. Beispiele für Erdalkalihydride sind Magnesiumhydrid und Calciumhydrid. Bevorzugt sind Alkalimetallhydride, wie z.B. Lithiumhydrid, Natriumhydrid oder Kaliumhydrid, insbesondere Natriumhydrid und Kaliumhydrid, besonders bevorzugt Natriumhydrid.

Geignet sind auch Alkoholate, wie z.B. $C_{1-6}$-(Erd)Alkalimetallalkoholate. Beispiele dafür sind $LiOCH_3$, $NaOCH_3$, $KOCH_3$, $LiOC_2H_5$, $NaOC_2H_5$, $KOC_2H_5$, KOtert-$C_4H_9$, $Ca(OC_2H_5)_2$, Mg(Otert-$C_5H_{11}$)$_2$. Bevorzugt werden Alkalimetall-$C_{1-6}$-alkoholate, insbesondere Na-Alkoholate, z.B. $NaOCH_3$, $NaOC_2H_5$ und NaOtert-$C_4H_9$, insbesondere $NaOCH_3$ und NaOtert-$C_4H_9$, verwendet.

Die Reaktionszeiten des oben beschriebenen Schrittes (D) (Claisen-Kondensation) können in weiten Grenzen schwanken, liegen im allgemeinen aber zwischen 0,5 und 5 Stunden.

Schritt (D) wird zweckmäßig in unter den Reaktionsbedingungen inerten organischen Lösungsmitteln durchgeführt. Als solche kommen beispielsweise lineare oder cyclische Ether, aliphatische oder aromatische Kohlenwasserstoffe oder cyclische oder lineare Amide in Frage.

Lineare oder cyclische Ether können beispielsweise Mono-, Di- Tri- oder Polyether sein. Beispiele dafür sind: Diethylether, Düsopropylether, Methyl-tert-butylether, Dibutylether, Ethylenglykoldimethylether, Diethylenglykoldimethylether, Tetrahydrofuran, Tetrahydropyran, Dioxan oder Dioxolan. Bevorzugt sind cyclische Ether und höhere lineare Ether (z.B. ab 5 C-Atomen), insbesondere Dioxan, Diethylenglykoldimethylether, Tetrahydrofuran oder Methyl-tert-butylether. Es können auch Mischungen dieser Lösungsmittel verwendet werden.

Als aliphatische Kohlenwasserstoffe werden beispielsweise Pentan, Hexan, Heptan, Octan, Cyclohexan, Decalin, Petrolether oder Mischungen davon verwendet. Zweckmäßig verwendete aromatische Kohlenwasserstoffe sind beispielsweise Benzol, Toluol oder Xylol, bevorzugt ist Toluol.

Als cyclisches oder lineares Amid kommt z.B. N-Methylpyrrolidon in Frage.

Bevorzugt werden als inerte organische Lösungsmittel Dioxan, Tetrahydrofuran, Diethylenglykoldimethylether, Methyl-tert-butylether, Toluol oder N-Methylpyrrolidon verwendet.

Daraus ergibt sich, daß die für die Stufen (A), (B), (C1) und (C2) bevorzugten Lösungsmittel (ausgenommen DMF) auch für den Schritt (D) sehr geeignet sind, was eine entscheidende Vereinfachung des Gesamtverfahrens mit sich bringt. Zwar kann der Ester ohne weiteres nach Schritt (C2) isoliert werden; bevorzugt aber kann auf eine Isolierung verzichtet werden. Man kann das Lösungsmittel durch Destillation entfernen und es durch ein anderes ersetzen. Besonders vorteilhaft ist es jedoch, die Claisen Kondensation in dem bereits in den Schritten (A) bis (C) verwendeten Lösungsmittel durchzuführen.

Von Bedeutung ist auch die Möglichkeit einer vereinfachten Aufarbeitung nach der Reaktion (D). Sie besteht darin, daß das aus der Reaktionslösung ausfallende Alkali- oder Erdalkalimetallsalz des Diketons direkt aus der Lösung abfiltriert und gewaschen wird, und danach durch Hydrolyse mit einer verdünnten Säure das reine Diketon erhalten wird. Als Säuren kommen beispielsweise Essigsäure, Ameisensäure, Phosphorsäure, Salzsäure oder Schwefelsäure in Frage, bevorzugt sind Salzsäure und Schwefelsäure.

Die Säuren der Formel I ($R_3$ = H) finden z.B auch als Korrosionsinhibitoren und Verschleißschutzadditive für Schmierstoffe und andere funktionelle Flüssigkeiten Verwendung (US-A-2,223,129, US-A-2,474,604). Diese Stoffe sind in D. Klamann,"Schmierstoffe und verwandte Produkte", Verlag Chemie, Weinheim 1982, ausführlich beschrieben.

Die bei dem erfindungsgemäßen Verfahren als Ausgangsstoffe eingesetzten Mercaptane und Lactone können, sofern nicht kommerziell erhältlich, nach bekannten Verfahren hergestellt werden.

Wie bereits erwähnt, stellen die erfindungsgemäß herstellbaren linearen 1,3-Diketone der Formel III wertvolle Costabilisatoren für chlorhaltige Polymerisate, die gegen schädigenden Einfluß von Wärme und/oder Licht geschützt werden müssen, dar (siehe z.B. EP-A-0 307 358). Es besteht daher ein Interesse daran, diese Diketone in möglichst einfachen Verfahren mit wenig Energieaufwand in hohen Ausbeuten herzustellen. Einen Schritt in diese gewünschte Richtung bildet die erleichterte Zugänglichkeit der für die Herstellung solcher schwefelhaltiger Diketone benötigten Ester durch das erfindungsgemäße Verfahren..

Die folgenden Ausführungsbeispiele erläutern die Erfindung näher, ohne sie jedoch zu beschränken. Sofern nicht anders angegeben, beziehen sich Teile- und Prozentangaben auf das Gewicht.

Beispiel 1

$$t\text{-}C_4H_9\text{-}S\text{-}(CH_2)_3\text{-}CO\text{-}O\text{-}CH_3$$

Zu 33,5 g Natriummethylat (97,5%ig) und 150 g N-Methylpyrrolidon werden unter Rühren 54 g tert-Butylmercaptan zugetropft. Anschließend wird das Gemisch auf 100°C erwärmt und innerhalb 30 min eine Lösung von 57 g γ-Butyrolacton in 150 g N-Methylpyrrolidon zudosiert. Es wird 60 Minuten bei 105 - 110°C gerührt, auf 90°C abgekühlt und dann eine Mischung von 43 g Schwefelsäure und 150 g Methanol unter Rückfluß eingetropft. Die Reaktionsmischung wird weitere 4 h am Rückfluß gerührt, mit 20 g Natriumhydrogencarbonat versetzt und das überschüssige Methanol abdestilliert. Nach dem Abkühlen auf 20°C wird die Mischung in 600 ml Wasser eingerührt und die wäßrige Phase 3 mal mit je 100 ml Diethylether extrahiert. Die mit der organischen Phase vereinigten Extrakte werden mit Wasser, Natriumhydrogencarbonatlösung und Wasser gewaschen, über Natriumsulfat getrocknet, am Rotationsverdampfer eingeengt und der Rückstand fraktioniert destilliert

Als Hauptfraktion gewinnt man 104 g (91% d.Th.) einer farblosen Flüssigkeit mit einem Siedepunkt von 98-102°C(16 Torr = 0,021 bar) und einem Brechungsindex $n_D^{20}$von 1,4605.

Beispiele 2-16

In Analogie zu Beispiel 1 werden unter Verwendung der äquivalenten Mengen des jeweiligen Mercaptans die in der nachfolgenden Tabelle I aufgeführten Verbindungen der Formel I erhalten. Das eingesetzte Lösungsmittel sowie die Reaktionstemperatur in der Stufe (B) sind jeweils angegeben.

Tabelle I:

| Nr. | Formel | Lösungsmittel | Temperatur | Siedepunkt [°C/bar] | $n_D^{20}$ |
|---|---|---|---|---|---|
| 2 | $t\text{-}C_9H_{19}\text{-}S\text{-}(CH_2)_3\text{-}CO\text{-}O\text{-}CH_3$ | N-Methylpyrrolidon | 105°C | 97-99°C/0.15 | 1,4705 |
| 3 | " | Dibutylether | 102°C | | |
| 4 | " | Diglyme | | | |
| 5 | $n\text{-}C_{12}H_{25}\text{-}S\text{-}CH(CH_3)\text{-}(CH_2)_2\text{-}COOCH_3$* | N-Methylpyrrolidon | 105°C | 125-127°C/0.14 | 1,4650 |
| 6 | $n\text{-}C_{12}H_{25}\text{-}S\text{-}(CH_2)_3\text{-}CO\text{-}O\text{-}CH_3$ | N-Methylpyrrolidon | 105°C | 132-134°C/0.2 | 1,4652 |
| 7 | " | Dibutylether | 100°C | " | " |
| 8 | " | Dioxan | 92°C | " | " |
| 9 | " | Diglyme | 105°C | " | " |
| 10 | " | Isomere Xylole | 97°C | " | " |
| 11 | " | p-Xylol | 94°C | " | " |
| 12 | " | o-Xylol | 95°C | " | " |
| 13 | " | DMSO | 110°C | " | " |
| 14 | $t\text{-}C_{12}H_{25}\text{-}S\text{-}(CH_2)_3\text{-}CO\text{-}O\text{-}CH_3$ | N-Methylpyrrolidon | 105°C | 120-125°C/0.08 | 1,4750 |
| 15 | 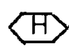 $\text{-}S\text{-}(CH_2)_3\text{-}CO\text{-}O\text{-}CH_3$ | N-Metyhlpyrrolidon | 105°C | 95-98°C/0.12 | 1,4894 |
| 16 | $Ph\text{-}S\text{-}(CH_2)_3\text{-}CO\text{-}O\text{-}CH_3$† | N-Methylpyrrolidon | 115°C | 101-104°C/0.08 | 1,5448 |

* Hier wurde an Stelle von γ-Butyrolacton α-Methyl-γ-butyrolacton eingesetzt.
† Ph bedeutet Phenyl

Beispiel 17

$n\text{-}C_{12}H_{25}\text{-}S\text{-}(CH_2)_3\text{-}CO\text{-}OH$

Zu einer Mischung von 23,3 g Natriummethylat (97%ig) und 100 g N-Methylpyrrolidon werden bei 80°C 81 g n-Dodecylmercaptan unter Rühren zugetropft. Es wird auf 105°C erwärmt und innerhalb 40 min werden 36,2 g γ-Butyrolacton, gelöst in 70 g N-Methylpyrrolidon zugetropft. Nach 60 minütigem Rühren bei 105°C wird auf 20°C abgekühlt und mit einer Mischung von 29g $H_2SO_4$ und 450 g Wasser versetzt. Die ausgefallene Säure wird abgesaugt, 2 mal mit je 100 ml Wasser gewaschen und bis zur Gewichtskonstanz getrocknet.

Ausbeute: 114,1g (98% d.Th.), farblose Kristalle Smp. 50-52°C

Beispiel 18

$t\text{-}C_{12}H_{25}\text{-}S\text{-}(CH_2)_3\text{-}CO\text{-}OH$

Analog zu den in Beispiel 17 angeführten Bedingungen wird statt n-Dodecylmercaptan t-Dodecylmercaptan eingesetzt. Nach dem Ansäuern mit verdünnter Schwefelsäure und Zugabe von weiteren 1,21 Wasser wird dreimal mit je 100 ml Diethylether extrahiert und die vereinigten organischen Phasen werden mit Wasser gewaschen. Nach Trocknen über Natriumsulfat wird am Rotationsverdampfer auf Rückstand eingeengt.
Ausbeute: 106,1g (92% d.Th.), rotbraune, viskose Flüssigkeit, $n_D^{20}$: 1,4818

## Patentansprüche

1. Verfahren zur Herstellung von Alkylthio- oder Arylthiocarbonsäuren bzw. deren Alkylestern der Formel I

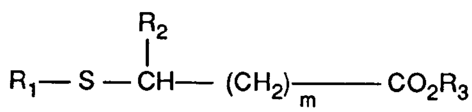

(I) , worin

$R_1$ $C_{1-20}$Alkyl oder mit $C_{1-4}$Alkoxy oder $C_{1-4}$Alkylthio substituiertes $C_{1-20}$Alkyl, $C_{5-12}$Cycloalkyl, Phenyl, mit $C_{1-4}$Alkyl oder $C_{1-4}$Alkoxy substituiertes Phenyl oder Phenyl-$C_{1-4}$Alkyl bedeutet,
$R_2$ Wasserstoff, $C_{1-12}$Alkyl oder mit $C_{1-4}$Alkoxy oder $C_{1-4}$Alkylthio substituiertes $C_{1-12}$Alkyl, Phenyl oder mit $C_{1-4}$Alkyl oder $C_{1-4}$Alkoxy substituiertes Phenyl ist, $R_3$ Wasserstoff oder $C_{1-0}$Alkyl und m eine Zahl von 1 bis 4 bedeuten, dadurch gekennzeichnet, daß
(A) ein Mercaptan $R_1SH$ in einem aprotischen Lösungsmittel mit Siedepunkt oberhalb 80°C mittels eines Alkalimetall-$C_{1-4}$-alkoholats oder -hydroxids als Base in sein Alkalisalz übergeführt wird,
(B) mit einem cyclischen, 4 bis 7 gliedrigen Lacton der Formel II

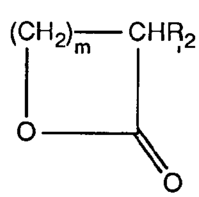

(II)

umgesetzt wird,
und das entstandene Salz
(C1) durch Ansäuern mit Mineralsäuren in eine Säure der Formel I ($R_3$ = H) übergeführt und isoliert wird bzw.
(C2) mit einem Alkohol $R_3OH$ zum Ester der Formel I ($R_3$ = $C_{1-0}$Alkyl) verestert wird, wobei alle Stufen ohne Isolierung von Zwischenprodukten im in Stufe (A) eingesetzten Lösungsmittel durchgeführt werden.

2. Verfahren nach Anspruch 1 zur Herstellung von Verbindungen der Formel I, worin $R_1$ $C_9$-$C_{12}$-Alkyl, $R_2$ Wasserstoff, $R_3$ Methyl sind und m die Zahl 3 bedeutet.

3. Verfahren nach Anspruch 1 zur Herstellung von Verbindungen der Formel I, worin $R_1$ $C_{12}$-Alkyl ist, $R_2$ und

$R_3$ Wasserstoff bedeuten und m die Zahl 3 ist.

**4.** Verfahren nach Anspruch 1, dadurch gekennzeichnet, daß es in einem Lösungsmittel, das die anfallenden Salze in ausreichender Menge löst, durchgeführt wird.

**5.** Verfahren nach Anspruch 1, dadurch gekennzeichnet, daß es in einem linearen oder cyclischen Ether, einem linearen oder cyclischen Amid oder einem aromatischen Kohlenwasserstoff als Lösungsmittel oder in Mischungen solcher Lösungsmittel durchgeführt wird.

**6.** Verfahren nach Anspruch 5, dadurch gekennzeichnet, daß es in einem der Lösungsmittel N-Methylpyrrolidon, Dibutylether, Dioxan, Diglyme, DMF, Xylol oder Mesitylen durchgeführt wird.

**7.** Verfahren nach Anspruch 1, dadurch gekennzeichnet, daß die Base in Stufe (A) NaOH, KOH, Natrium- oder Kaliummethylat, -ethylat oder -butylat ist.

**8.** Verfahren nach Anspruch 1, worin $R_1$ $C_{1-18}$Alkyl, $C_{5-7}$Cycloalkyl, Phenyl oder Benzyl ist, $R_2$ Wasserstoff oder $C_{1-4}$Alkyl ist und m für 2 steht.

**9.** Verfahren nach Anspruch 1, wobei $R_3$ $C_{1-8}$Alkyl ist.

**10.** Verfahren nach Anspruch 1, wobei $R_3$ H ist.

**11.** Verfahren nach Anspruch 9, dadurch gekennzeichnet, daß die Veresterung (C2) unter sauren Bedingungen durchgeführt wird.

**12.** Verfahren nach Anspruch 11, dadurch gekennzeichnet, daß in Stufe (C2) als Veresterungskatalysator Schwefelsäure benutzt wird.

**13.** Verfahren nach Anspruch 11, dadurch gekennzeichnet daß in Stufe (C2) die Reaktionsmischung nach der Veresterung mit geeigneten Basen neutralisiert wird und das Produkt durch Extraktion und Abziehen des Extraktionsmittels isoliert wird.

**14.** Verfahren nach Anspruch 13, dadurch gekennzeichnet daß die Base Natrium- oder Kaliumcarbonat oder -hydrogencarbonat ist.

**15.** Verfahren zur Herstellung von β-Diketonen der Formel III

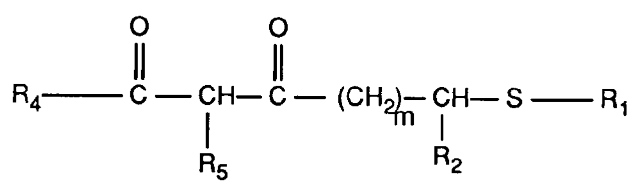

(III),

durch (D) Claisen-Kondensation eines Esters der Formel I, hergestellt nach dem Verfahren gemäß Anspruch 7, mit einem Keton der Formel IV

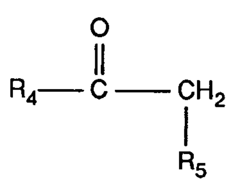

(IV),

worin $R_1$, $R_2$ und m die in Anspruch 1 angegebenen Bedeutungen haben, $R_4$ für $C_1$-$C_{20}$-Alkyl, Phenyl, durch Halogen, Hydroxy, $NO_2$, $C_1$-$C_4$-Alkyl und/oder $C_1$-$C_4$-Alkoxy substituiertes Phenyl, $C_7$-$C_9$-Phenylalkyl oder für einen Rest der Formel V steht,

$$-A-X-R_6 \qquad (V)$$

wobei

A $C_1$-$C_{12}$-Alkylen, Phenylen, durch Halogen, Hydroxy, $NO_2$, $C_1$-$C_4$-Alkyl und/oder $C_1$-$C_4$-Alkoxy substituiertes Phenylen oder durch Hydroxy, Halogen oder/und Alkoxy substituiertes $C_1$-$C_{12}$-Alkylen bedeutet,
X für Sauerstoff oder Schwefel steht und
$R_6$ Wasserstoff, $C_1$-$C_{18}$-Alkyl, Phenyl, durch Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl bedeutet und
$R_5$ Wasserstoff, $C_1$-$C_{20}$-Alkyl, Phenyl, durch Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl darstellt, in Gegenwart einer Base.

**16.** Verfahren nach Anspruch 15, dadurch gekennzeichnet, daß die Kondensation (D) in Gegenwart eines Alkali- oder Erdalkalimetallhydrids oder $C_{1-6}$-Alkali- oder Erdalkalimetallakoholats als Base erfolgt.

**17.** Verfahren nach Anspruch 15, worin
$R_4$ $C_1$-$C_{18}$-Alkyl, Phenyl, ($C_1$-$C_4$-Alkyl)-phenyl oder einen Rest der Formel V bedeutet,
A für $C_1$-$C_6$-Alkylen steht,
$R_6$ Wasserstoff, $C_1$-$C_{18}$-Alkyl, Phenyl oder ($C_1$-$C_4$-Alkyl)-phenyl darstellt und
$R_5$ für Wasserstoff und $C_1$-$C_4$-Alkyl steht.

Europäisches Patentamt

# EUROPÄISCHER RECHERCHENBERICHT

Nummer der Anmeldung

EP 92 81 0057
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 966 731 (Y. CHOU)<br>* Spalte 3, Zeile 33 - Spalte 4, Zeile 37 *<br>--- | 1,3,4,7, 8,10 | C07C319/14<br>C07C319/20 |
| X | CHEMICAL ABSTRACTS, vol. 113, no. 7, 13. August 1990, Columbus, Ohio, US; abstract no. 58500F, S. IKESU ET AL.: 'PREPARATION OF 4-ALKYLTHIOBUTYRIC ACIDS AND ANALOGS AS PHOTOGRAPHIC ADDITIVES.'<br>Seite 640 ;Spalte 2 ;<br>* Zusammenfassung *<br>& JP-A-02 072 156 (KONICA CO.) 12. März 1990<br>--- | 1,3,4,7, 8,10 | |
| D,X | EP-A-0 307 358 (CIBA-GEIGY AG)<br>* Seite 8, Zeile 9 - Zeile 29 *<br>* Seite 13; Beispiele 19,20 *<br>--- | 15-17 | |
| D,A | EP-A-0 245 231 (MONSANTO COMPANY)<br>* Spalte 6, Zeile 37 - Spalte 7, Zeile 63 *<br>--- | 1,3-8,10 | |
| D,A | THE JOURNAL OF ORGANIC CHEMISTRY<br>Bd. 46, Nr. 25, 4. Dezember 1981, Seiten 5163 - 5166;<br>M. NODE ET AL.: 'HARD ACID AND SOFT NUCLEOPHILE SYSTEMS. 5. RING-OPENING REACTION OF LACTONES TO w-ALKYLTHIO OR w-ARYLTHIO CARBOXYLIC ACIDS WITH ALUMINUM HALIDE AND THIOL.'<br>* das ganze Dokument *<br>--- | 1,3-8,10 | **RECHERCHIERTE SACHGEBIETE (Int. Cl.5)**<br>C07C |
| D,A | FR-A-2 137 678 (MOBIL OIL CORPORATION)<br>* Seite 5 - Seite 6; Beispiele 1,2,4 *<br>--- | 1,3-8,10 | |
| A | DE-C-816 544 (KNOLL A.G.)<br>* Seite 1, Spalte 1, Zeile 10 - Seite 1, Spalte 2, Zeile 17 *<br>* Seite 2; Beispiel 1 *<br>---<br>-/-- | 1,3-8,10 | |

Der vorliegende Recherchenbericht wurde für alle Patentansprüche erstellt

| Recherchenort | Abschlußdatum der Recherche | Prüfer |
|---|---|---|
| DEN HAAG | 20 MAI 1992 | FINK D.G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 92 81 0057
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | EP-A-0 454 624 (CIBA-GEIGY AG)<br>* Seite 7; Beispiel 4 *<br>----- | 15-17 | |

RECHERCHIERTE SACHGEBIETE (Int. Cl.5 )

Der vorliegende Recherchenbericht wurde für alle Patentansprüche erstellt

| Recherchenort | Abschlußdatum der Recherche | Prüfer |
|---|---|---|
| DEN HAAG | 20 MAI 1992 | FINK D.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)